# EUROPÄISCHE PATENTSCHRIFT

(11) **EP 2 174 587 B1**
(45) Veröffentlichungstag und Bekanntmachung des Hinweises auf die Patenterteilung: **04.05.2011**
(21) Anmeldenummer: 08165704.1
(22) Anmeldetag: 02.10.2008
(51) Int. Cl.: A61B 5/00, A61B 17/34

(54) **Implantationsvorrichtung für Metabolitsensoren**
Implantation device for metabolite sensors
Dispositif d'implantation pour capteurs de métabolites

(43) Veröffentlichungstag der Anmeldung: 14.04.2010
(73) Patentinhaber: EyeSense AG, 4051 Basel (CH)
(72) Erfinder: Müller, Achim, 63762, Grossostheim (DE); Herbrechtsmeier, Peter, 61462, Königstein (DE)
(74) Vertreter: Stößel, Matthias

(56) Entgegenhaltungen:
- WO-A-94/04217
- WO-A-2007/058921
- US-A1- 2008 139 903
- MCSHANE M J ET AL: "GLUCOSE MONITORING USING IMPLANTED FLUORECENT MICROSPHERES WORKING TOWARD A MININALLY INVASIVE MEANS FOR DIABETICS TO BETTER MONITOR GLUCOSE LEVELS" IEEE ENGINEERING IN MEDICINE AND BIOLOGY MAGAZINE, IEEE SERVICE CENTER, PISACATAWAY, NJ, US, Bd. 19, Nr. 6, 1. November 2000 (2000-11-01), Seiten 36-45, XP000975447 ISSN: 0739-5175

## Beschreibung

Die Erfindung betrifft eine Implantationsvorrichtung zur Implantation eines Sensorelements zur Erfassung mindestens eines Analyten. Derartige Sensorelemente werden insbesondere für die Bestimmung mindestens einer Metabolitkonzentration in einer Körperflüssigkeit und/oder einem Körpergewebe eingesetzt. Derartige Metaboliten können beispielsweise, jedoch nicht ausschließlich, Blutglucose, Lactat, Cholesterin oder andere Arten von Analyten bzw. Metaboliten umfassen. Alternativ oder zusätzlich lässt sich das Sensorelement jedoch grundsätzlich auch in anderen Bereichen der Analytik einsetzen, beispielsweise im Bereich der chemischen Analytik, insbesondere der in-situ-Analytik, der Prozessüberwachung oder in ähnlichen Bereichen.

Viele herkömmliche Systeme zur Bestimmung von Analyt- bzw. Metabolitkonzentrationen basieren in vielen Fällen auf der Generierung einer Probe einer Körperflüssigkeit, wie beispielsweise eines Blutstropfens, welche anschließend mittels eines geeigneten Messgerätes auf ihren Analytgehalt untersucht wird. Hierbei können beispielsweise optische und/oder elektrochemische Messverfahren zum Einsatz kommen.

Um die mit der häufigen Generierung von Blutproben verbundenen Unannehmlichkeiten der Patienten zu verringern, wurden verschiedene nicht-invasive oder minimal-invasive Technologien zur Messung von Analytkonzentrationen entwickelt. Im Folgenden wird dabei ohne Beschränkung des Schutzumfangs der Erfindung auf die Bestimmung der Blutglucosekonzentration eingegangen, wobei jedoch naturgemäß alternativ oder zusätzlich auch andere Arten von Analyten bzw. Metaboliten nachweisbar sind.

Die invasiven Technologien zur Bestimmung der Analytkonzentration basieren üblicherweise auf in eine Körpergewebe und/oder eine Körperflüssigkeit implantierbaren Sensoren, welche auf optische und/oder elektrochemische Weise die Analytkonzentration bestimmen können. Optische Systeme verwenden dabei in der Regel mindestens ein Sensormaterial, welches bei Anwesenheit eines oder mehrerer bestimmter Analyten mindestens eine optisch messbare Eigenschaft ändert. Diese optisch messbare Eigenschaft kann auf verschiedenste Weisen ausgestaltet sein, wobei viele verschiedene Verfahren, Sensormaterialien und Messvorrichtungen aus dem Stand der Technik bekannt sind. Sämtliche dieser bekannten Sensormaterialien lassen sich grundsätzlich auch im Rahmen der vorliegenden Erfindung einsetzen. Auch auf elektrochemischen Messverfahren beruhende Sensorelemente sind jedoch im Rahmen der vorliegenden Erfindung mit der Implantationsvorrichtung einsetzbar.

Beispielsweise beschreibt WO 01/13783 einen Okularsensor für Glucose, welcher als Augenlinse ausgestaltet ist. Der Okularsensor umfasst als Sensormaterial einen Glucoserezeptor, welcher mit einem ersten Fluoreszenzlabel markiert ist, und einen Glucose-Konkurrenten, welcher mit einem zweiten Fluoreszenzlabel ("Donor") markiert ist. Die beiden Fluoreszenzlabel sind derart gewählt, dass, wenn der Konkurrent an den Rezeptor gebunden ist, die Fluoreszenz des zweiten Fluoreszenzlabels aufgrund eines resonanten Fluoreszenz-Energietransfers gelöscht wird (Quenching). Durch Überwachen der Veränderung der Fluoreszenzintensität bei einer Wellenlänge um das Fluoreszenzmaximum des quenchbaren Fluoreszenzlabels kann der Anteil des Fluoreszenz-markierten Konkurrenten gemessen werden, welcher durch die Glucose verdrängt worden ist. Auf diese Weise kann die Glucosekonzentration in der Augenflüssigkeit bestimmt werden. Die Messung kann wiederum genutzt werden, um daraus auf die Blutglucosekonzentration zu schließen. Auch andere Arten von Nachweisen sind denkbar und dem Fachmann geläufig, beispielsweise ein Fluoreszenznachweis des ersten Fluoreszenzlabels.

WO 02/087429 beschreibt ein Fluoreszenz-Photometer, mittels dessen Blutglucosekonzentrationen durch Messung der Glucosekonzentrationen an der Augenflüssigkeit bestimmt werden können. Die dargestellte Vorrichtung ist in der Lage, gleichzeitig zwei Fluoreszenzintensitäten bei verschiedenen Wellenlängen zu messen.

Zur Ein- und Auskopplung optischer signale in bzw. aus den Sensorelementen bestehen verschiedene Konzepte, je nach der Gewebeart des Gewebes, in welches das Sensorelement implantiert ist. Bei den in WO 01/13783 und in WO 02/087429 beschriebenen Sensorelementen sind die Gewebeschichten, welche den implantierten Sensor bedecken, in der Regel im Augenbereich transparent und ermöglichen somit ein Ein- bzw. Auskoppeln von Lichtsignalen.

Für nicht-transparente Gewebearten beschreibt beispielsweise WO 2005/054831 A1 ein Sensorelement zur Bestimmung einer Glucosekonzentration, bei welchem ein optischer Lichtwellenleiter verwendet wird. Am distalen Ende des Lichtwellenleiters wird ein Sensorelement auf denselben aufgebracht, welches ein bindendes Protein umfasst, das mit mindestens einem Ziel-Analyt binden kann. Das Sensorelement umfasst weiterhin mindestens ein Reporter-Gruppe, welche einer Lumineszenzänderung unterliegt, wenn sich die Analytkonzentrationen ändern. Optional umfasst das Sensorelement Referenzgruppen mit Lumineszenzeigenschaften, die sich im Wesentlichen nicht ändern, wenn sich die Analytkonzentrationen ändern.

Auch US 7,226,414 B2 beschreibt eine Glucose-Sensorvorrichtung zur Implantation innerhalb des subkutanen Gewebes eines tierischen Körpers. Ein Sensormaterial ist in einer ersten Kammer angeordnet, wobei Glucose aus dem Körpergewebe in die erste Kammer eintreten kann. Das Sensorelement umfasst weiterhin eine Referenzkammer mit einer Referenzlösung. Zum Ankoppeln eines Auslesegerätes wird wieder die Verwendung von Lichtwellenleiter-Fasern vorgeschlagen, welche ein Nachweisgerät mit den Kammern verbinden.

In US 2007/0122829 A1 werden ein System, eine Vorrichtung und ein Verfahren zur Messung der Konzentration eines Analyten in einer Flüssigkeit oder einer Matrix vorgeschlagen. Ein thermodynamisch stabilisierter, Analyt-bindender Ligand wird vorgeschlagen. Auch in diesem Fall wird wiederum die Verwendung eines an ein Sensorelement angekoppelten separaten Lichtwellenleiters in Form einer Faser vorgeschlagen, welche ein Nachweisgerät mit einem implantierten Sensorelement verbindet.

Eine Herausforderung bei implantierbaren Sensorelementen besteht insbesondere in einer gleichmäßigen, reproduzierbaren und dennoch möglichst schmerzfreien Implantation der Sensorelemente in das Körpergewebe. Insbesondere bei Sensorelementen mit optischer Kopplung, welche ganz oder teilweise von einer Hautpartie überdeckt sind, jedoch auch beispielsweise bei elektrochemischen Sensorelementen, wirken sich die Implantationstiefe und die Sensorposition signifikant auf die Signalqualität aus. Wünschenswert ist zudem, um eine möglichst schmerzfreie Implantation und anschließend eine möglichst schmerzfreie Entfernung der Sensorelemente zu gewährleisten, eine möglichst minimal-invasive Implantationstechnologie.

In WO94/04217 wird eine Vorrichtung zur gleichzeitigen Injektion von einer Flüssigkeit und einem Transponder in ein tierisches Gewebe beschrieben. Diese Vorrichtung ermöglicht anhand eines einzigen Einstichs beide Komponenten zu injizieren.

Es ist daher eine Aufgabe der vorliegenden Erfindung, eine Implantationsvorrichtung zur Implantation eines Sensorelements in ein Körpergewebe bereitzustellen, welche die oben beschriebenen Schwierigkeiten überwindet. Insbesondere soll die Implantationsvorrichtung eine reproduzierbare Implantation von Sensorelementen ermöglichen, sowie eine möglichst schmerzfreie Einbettung der Sensorelemente in das Körpergewebe gewährleisten.

Diese Aufgabe wird durch eine Implantationsvorrichtung mit den Merkmalen des unabhängigen Anspruchs gelöst. Vorteilhafte Weiterbildungen der Erfindung, welche einzeln oder in Kombination realisierbar sind, sind in den Unteransprüchen dargestellt.

Die Implantationsvorrichtung kann grundsätzlich genutzt werden, um Sensorelemente zur Erfassung mindestens eines Analyten in einer Körperflüssigkeit und einem Körpergewebe in das Körpergewebe zu implantieren. Insbesondere können die Sensorelemente ein oder mehrere der oben beschriebenen Sensorelemente umfassen. Die Sensorelemente können insbesondere zur Bestimmung mindestens einer Metabolitkonzentration in einer Körperflüssigkeit eingesetzt werden. Als mögliche Beispiele von Analyten kann auf die obige Beschreibung des Standes der Technik verwiesen werden. Unter dem Begriff "Erfassung" kann dabei eine quantitative und/oder qualitative Bestimmung einer Analytkonzentration verstanden werden, das heißt eine Bestimmung der Menge und/oder Konzentration des Analyten in der Körperflüssigkeit und/oder die Beantwortung der Frage, ob der Analyt überhaupt in der Körperflüssigkeit enthalten ist.

Die Implantationsvorrichtung umfasst mindestens eine Kanüle zum Durchdringen einer Hautoberfläche eines Patienten. Unter einer Kanüle ist im Rahmen der vorliegenden Erfindung ein im Wesentlichen rohrförmiges Gebilde zu verstehen, welches starr oder flexibel ausgestaltet sein kann, und welches ein Innenlumen aufweist. Dieses Innenlumen kann einen konstanten oder einen variablen Querschnitt umfassen. Anstelle eines einzelnen Innenlumens können auch Kanülen verwendet werden, welche mehrere Innenlumina aufweisen, so dass sich beispielsweise auch mehrschichtige Sensorelemente erzeugen bzw. implantieren lassen. So kann die Kanüle beispielsweise mehrere ringförmig umeinander angeordnete Lumina umfassen, in welchen beispielsweise unterschiedliche Komponenten der Sensorelemente aufgenommen werden können. Auch benachbarte Anordnungen mehrerer Lumina sind denkbar.

Zum Durchdringen der Hautoberfläche kann beispielsweise vor Verwendung der Implantationsvorrichtung ein Schnitt in der Hautoberfläche gesetzt werden. Besonders bevorzugt ist es jedoch, wenn die Kanüle selbst ein Element zum Durchdringen der Hautoberfläche aufweist, beispielsweise eine Kanülenspitze und/oder ein scharfkantiges Element, welches eingerichtet ist, um die Hautoberfläche zu perforieren.

Die Kanüle soll mindestens einen Aufnahmebereich zum Aufnehmend des Sensorelements aufweisen. Beispielsweise kann dieser Aufnahmebereich einen Bereich zwischen einer Kanülenspitze, welche zuerst in die Hautoberfläche eingeführt wird, und einer Verengung innerhalb der Kanüle umfassen, beispielsweise einer Verengung innerhalb des Innenlumens der Kanüle. Es sind jedoch auch Ausführungsformen denkbar, bei welchen der Aufnahmebereich sich vom übrigen Innenlumen der Kanüle nicht unterscheidet. Der Aufnahmebereich kann insbesondere kreiszylinderförmig ausgestaltet sein, um kreiszylinderförmige Sensorelemente, also Sensorelemente mit einem Kreisquerschnitt und einer lang gestreckten Form, aufzunehmen. Beispielsweise kann der Aufnahmebereich einen Durchmesser zwischen 100 µm und einem 1 mm aufweisen, wobei besonders bevorzugt Durchmesser im Bereich von 200 bis 500 µm sind. Für die Länge des Aufnahmebereichs und somit die Länge der Sensorelemente bieten sich beispielsweise Längen zwischen 1 mm und 8 mm, vorzugsweise zwischen 2 mm und 5 mm an.

Weiterhin weist die Implantationsvorrichtung mindestens einen mit der Kanüle verbundenen Hydraulikbehälter zum Aufnehmen eines Hydraulikfluids auf. Die Verbindung zwischen dem Hydraulikbehälter und der Kanüle kann beispielsweise starr erfolgen, so dass der Hydraulikbehälter und die Kanüle gemeinsam eine Implantationseinheit bilden. Insbesondere kann die Kanüle unmittelbar mit dem Hydraulikbehälter verbunden sein, wobei jedoch alternativ auch Verbindungen über Zwischenelemente möglich sind, beispielsweise einen oder mehrere Schläuche.

Der Hydraulikbehälter soll insbesondere in gewissem Rahmen druckfest ausgebildet sein. Als Hydraulikfluid bieten sich grundsätzlich Gase und/oder Flüssigkeiten an, wobei Flüssigkeiten bevorzugt sind. Das Hydraulikfluid soll vorzugsweise biokompatibel ausgestaltet sein. Besonders bevorzugt ist daher die Verwendung einer Kochsalzlösung, insbesondere einer physiologischen Kochsalzlösung, als Hydraulikfluid. Es können jedoch auch andere Arten von Fluiden verwendet werden sowie Mischungen bzw. Kombinationen verschiedener Fluide.

Die Implantationsvorrichtung weist weiterhin mindestens eine Druckerzeugungsvorrichtung auf. Diese Druckerzeugungsvorrichtung soll eingerichtet sein, um das Hydraulikfluid mit einem Druck, das heißt einem Überdruck und/oder einem Unterdruck, zu beaufschlagen. Auf diese Weise kann, indem das Hydraulikfluid in dem Hydraulikbehälter und somit in der Kanüle mit einem Überdruck beaufschlagt wird, das Sensorelement mittels dieses druckbeaufschlagten Hydraulikfluids aus der Kanüle in das Körpergewebe transferiert werden, beispielsweise hydraulisch in das Körpergewebe geschoben werden.

Die Druckerzeugungsvorrichtung zur Erzeugung des Über- bzw. Unterdrucks in dem Hydraulikbehälter kann insbesondere mindestens einen Druckkolben umfassen, welcher mit dem Hydraulikbehälter hydraulisch in Verbindung steht. Beispielsweise kann der Druckkolbcn ganz oder teilweise in dem Hydraulikbehälter aufgenommen sein. Alternativ oder zusätzlich kann der Druckkolben jedoch auch in einem anderen Element der Implantationsvorrichtung aufgenommen sein, beispielsweise in einem separaten Druckerzeuger, welcher hydraulisch mit dem Hydraulikbehälter in Verbindung steht, beispielsweise über eine Schlauch- oder Rohrverbindung. Auch andere Arten von Druckerzeugern sind jedoch grundsätzlich denkbar, beispielsweise Druckerzeuger mit einer separaten Druckquelle, beispielsweise einer Pumpe, einer externen Druckquelle oder ähnlichem.

Besonders bevorzugt ist es, wenn die Implantationseinheit, welche die Kanüle und den Hydraulikbehälter umfasst, relativ zu dem Druckkolben als Ganzes bewegbar ist. Beispielsweise kann die Implantationseinheit axial, das heißt entlang einer Achse der Implantationsvorrichtung, relativ zu dem Druckkolben bewegbar sein. Besonders bevorzugt ist es dabei, wenn der Druckkolben in seiner Position, beispielsweise in seiner Position relativ zur Hauptoberfläche des Patienten, fixierbar ist, so dass lediglich die Implantationseinheit relativ zum Druckkolben und somit auch relativ zur Hautoberfläche bewegt wird. Zu diesem Zweck kann der Druckkolben beispielsweise mit mindestens einer Auflagefläche zur Auflage auf der Hautoberfläche verbunden sein, beispielsweise über ein Kolbengestänge. Diese Auflagefläche kann ganz oder teilweise bauteilidentisch sein mit einer Vorrichtung, welche auch zur Einstellung und/oder Begrenzung der Implantationstiefe verbunden ist.

Eine derartige Vorrichtung zur Einstellung und/oder Begrenzung der Implantationstiefe, welche auch unabhängig von der Ausgestaltung des Druckerzeugers bzw. Druckkolbens vorgesehen sein kann, kann die Reproduzierbarkeit der Implantation des Sensorelements erheblich verbessern. Diese Reproduzierbarkeit ist jedoch, wie oben dargestellt, von entscheidender Bedeutung für die Signalqualität, insbesondere bei optischen Sensorelementen. Zu diesem Zweck wird daher vorgeschlagen, die Implantationsvorrichtung mit einer derartigen Vorrichtung zu versehen.

Insbesondere kann eine derartige Vorrichtung zur Einstellung und/oder Begrenzung der lmplantationstiefe einen Tiefenanschlag umfassen, beispielsweise einen Stopper, welcher eine Bewegung der Implantationseinheit relativ zur Hautoberfläche und somit einer Eindringtiefe der Kanüle in die Hautoberfläche begrenzt. So kann die Vorrichtung beispielsweise eine Auflagefläche zur Auflage auf der Hautoberfläche umfassen, welche somit eine Fixposition relativ zur Hautoberfläche bereitstellen kann. Insbesondere kann diese Auflagefläche eine breitflächige Auflagefläche sein, also eine Auflagefläche mit einer Größe von einigen zehn Quadratmillimetern bis hin zu einigen Quadratzentimetern. Die Auflagefläche kann beispielsweise die Kanüle bzw. die Kanülenspitze teilweise ringförmig (beispielsweise kreisringförmig) umgeben. Wie oben beschrieben, kann die Auflagefläche insbesondere mit dem Druckkolben verbunden sein, beispielsweise über ein Kolbengestänge.

Ist eine derartige Vorrichtung zur Einstellung und/oder Begrenzung der Implantationstiefe vorgesehen, so ist es besonders bevorzugt, wenn die Implantationseinheit, die den Hydraulikbehälter und die Kanüle umfasst, mit dieser Vorrichtung über mindestens ein Federelement verbunden ist. Dieses Federelement, welches beispielsweise als Spiralfederelement oder als Blattfederelement ausgestaltet sein kann, welches jedoch alternativ oder zusätzlich auch andere Arten elastischer Elemente umfassen kann, kann als Rückholfeder dienen und nach erfolgter Implantation ein Herausziehen der Kanüle aus dem Körpergewebe begünstigen. Insbesondere kann das Federelement wiederum mit der Auflagefläche verbunden sein und die Implantationseinheit mit einer Vorspannung relativ zu dieser Auflagefläche beaufschlagen bzw. von dieser Auflagefläche wegdrücken.

Der Hydraulikbehälter der Implantationsvorrichtung ist über mindestens eine Verbindung mit einem Hydraulikvorrat zur Aufnahme und Bereitstellung des Hydraulikfluids verbunden. Beispielsweise kann dieser Hydraulikvorrat einen Vorratstank umfassen. Die Verbindung kann beispielsweise eine oder mehrere Schlauchverbindungen umfassen, welche eine Bewegung der Implantationseinheit relativ zu dem Hydraulikvorrat tolerieren. Alternativ kann der Hydraulikvorrat jedoch auch ganz oder teilweise fest mit der Implantationseinheit verbunden sein, beispielsweise mit dem Hydraulikvorrat.

Die Verbindung umfasst mindestens ein Ventil. Besonders bevorzugt ist es, wenn dieses Ventil ein Rückschlagventil umfasst. So kann das Ventil beispielsweise eingerichtet sein, um bei einem Unterdruck in dem Hydraulikbehälter zu öffnen und ein Nachströmen von Hydraulikfluid in den Hydraulikbehälter zu ermöglichen.

Insbesondere letztere Ausgestaltung der Implantationsvorrichtung ermöglicht eine besonders einfache und reproduzierbare Implantation der Sensorelemente. So kann beispielsweise die Implantationseinheit mit dem Hydraulikbehälter und der Kanüle relativ zur Hautoberfläche abgesenkt werden, so dass die Kanülenspitze mit dem Aufnahmebereich in die Hautoberfläche eindringt. Die Druckerzeugungsvorrichtung kann derart eingerichtet sein, dass bei diesem Absenken ein Unterdruck in dem Hydraulikbehälter erzeugt wird. Dies kann beispielsweise dadurch erfolgen, dass, wie oben beschrieben, ein feststehender Druckkolben verwendet wird. Wird die Implantationseinheit relativ zu diesem Druckkolben gesenkt, so entsteht im Hydraulikbehälter ein Unterdruck. Über das Ventil, insbesondere das Rückschlagventil, kann dann Hydraulikfluid in den Hydraulikbehälter nachströmen. Wird anschließend, beispielsweise angetrieben durch die Rückholfeder bzw. das Federelement, die Implantationseinheit wieder angehoben und die Kanüle aus der Hautoberfläche herausgezogen, so steigt, bedingt durch die feste Position des Druckkolbens, der Druck in dem Hydraulikbehälter wieder an. Durch diesen Druckanstieg mit dem Herausziehen der Kanüle aus dem Gewebe wird dabei das Sensorelement aus dem Aufnahmebereich der Kanüle in das Gewebe gedrückt.

Diese Ausgestaltung der Implantation bietet den Vorteil, dass sich die Implantation auf eine Handhabung der Implantationseinheit und eine Steuerung der Position dieser Implantationseinheit beschränken kann. Eine separate Steuerung des Herausschiebens des Sensorelements aus dem Aufnahmebereich der Kanüle in das Gewebe ist nicht erforderlich, da dieses Herausschieben bzw. Transferieren in das Körpergewebe automatisch mittels des Hydraulikfluids erfolgt. Bevorzugt ist also die Implantationsbewegung allgemein derart mit dem Druckerzeuger gekoppelt, dass eine Implantationsbewegung automatisch den Transfer des Sensorelements aus der Kanüle in das Körpergewebe bewirkt.

Analog kann die Implantationsvorrichtung auch für eine Entfernung des Sensorelements aus dem Körpergewebe eingerichtet sein. So kann beispielsweise für ein Entfernen des Sensorelements eine dem Sensorelement entsprechende Kanüle durch die Hautoberfläche in das Körpergewebe eingeführt werden, so dass das Sensorelement ganz oder teilweise in die Öffnung der Kanüle eingeschoben wird. Die Druckerzeugungsvorrichtung kann entsprechend eingerichtet sein, um einen Unterdruck in dem Hydraulikfluid zu erzeugen, wodurch das Sensorelement in die Kanüle gesaugt werden kann. Beispielsweise kann zu diesem Zweck das Ventil, welches den Hydraulikvorrat und den Hydraulikbehälter verbindet, geschlossen werden, oder es kann ein umgekehrt wirkendes Rückschlagventil verwendet werden. Auf diese Weise kann beim Absenken der Implantationsvorrichtung, wobei das Sensorelement ganz oder teilweise in die Kanüle bzw. den Aufnahmebereich der Kanüle eingeschoben wird, ein Unterdruck in dem Hydraulikfluid erzeugt werden, mittels dessen das Sensorelement vollständig in die Kanüle eingesaugt wird. Auch eine andere Ausgestaltung der Drucksteuerung ist möglich, beispielsweise eine Drucksteuerung, bei welcher der Unterdruck erst beim anschließenden erneuten Anheben und damit Entfernen der Kanüle aus dem Körpergewebe erfolgt. Verschiedene Ausgestaltungen sind denkbar.

Um, insbesondere beim Entfernen des Sensorelements aus dem Körpergewebe, ein zu tiefes Eindringen des zu entfernenden Sensorelements in die Kanüle, insbesondere jenseits des Aufnahmebereichs der Kanüle, zu verhindern, kann die Kanüle, wie oben beschrieben, über mindestens eine Verengung verfügen. Diese Verengung, welche beispielsweise in Form einer Einschnürung des Innenlumens der Kanüle und/oder in Form einer konischen Verengung des Innenlumens der Kanüle ausgestaltet sein kann, verhindert ein weiteres Vordringen des Sensorelements in Richtung des Hydraulikbehälters.

In einer besonders bevorzugten Ausführungsform der Erfindung ist die Implantationsvorrichtung derart eingerichtet, dass das Sensorelement ganz oder teilweise unmittelbar in der Implantationsvorrichtung hergestellt werden kann. So können beispielsweise Sensorelemente vernetzbare Materialien umfassen, welche unmittelbar in der Kanüle vernetzt werden können. Beispielsweise können zu diesem Zweck vernetzbare Kunststoffe verwendet werden, insbesondere vernetzbare Hydrogele, welche beispielsweise durch eine photochemische Anregung vernetzt werden können. Zu diesem Zweck ist es besonders bevorzugt, wenn die Kanüle zumindest teilweise transparent für elektromagnetische Strahlung, insbesondere für Licht, ist. Insbesondere soll eine Transparenz für Licht im ultravioletten Spektralbereich vorliegen. Die Implantationsvorrichtung kann dementsprechend mindestens eine Lichtquelle zum Vernetzen des mindestens einen vernetzbaren Materials des Sensorelements in der Kanüle umfassen, beispielsweise eine Lichtquelle zur Erzeugung ultravioletter Strahlung.

Die vorgeschlagene Implantationsvorrichtung in einer oder mehreren der oben beschriebenen Ausführungsformen ermöglicht somit eine zuverlässige und höchst reproduzierbare Implantation von Sensorelementen der verschiedensten Bauarten. Die Implantation kann schmerzfrei und mit genau vorgebbarer Implantationstiefe in verschiedene Arten von Körpergewebe erfolgen, wobei beispielsweise die Implantationstiefe durch eine Veränderung einer Einstellung (beispielsweise mittels einer Stellschraube und/oder einer elektromechanischen Vorrichtung) der Implantationsvorrichtung realisierbar ist. Die Implantationsvorrichtung bzw. die Implantationseinheit kann manuell bewegbar sein. Alternativ oder zusätzlich kann jedoch auch eine automatische Positionierung bzw. ein automatischer Antrieb der Implantationseinheit vorgesehen sein, beispielsweise ein Antrieb mittels einer oder mehrerer Aktoren, welche einen Vortrieb und/oder Rücktrieb der Implantationseinheit, insbesondere der Kanüle, in das bzw. aus dem Körpergewebe bewirken. Die Implantation kann zum einen in undurchsichtiges Körpergewebe erfolgen, beispielsweise in Hautpartien, oder kann auch in transparente Gewebebereiche erfolgen, wie beispielsweise einen Gewebebereich am Auge. Insbesondere durch vorsehen einer entsprechenden Auflagefläche, welche an die körperlichen Gegebenheiten im Bereich der Implantationsstelle optimal angepasst sein kann, kann hierbei eine hohe Reproduzierbarkeit gewährleistet werden.

Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1A bis 1C: eine erfindungsgemäße Implantationsvorrichtung in verschiedenen Stadien eines Implantationsvorgangs; und
- Figur 2: ein in ein Körpergewebe implantiertes Sensorelement.

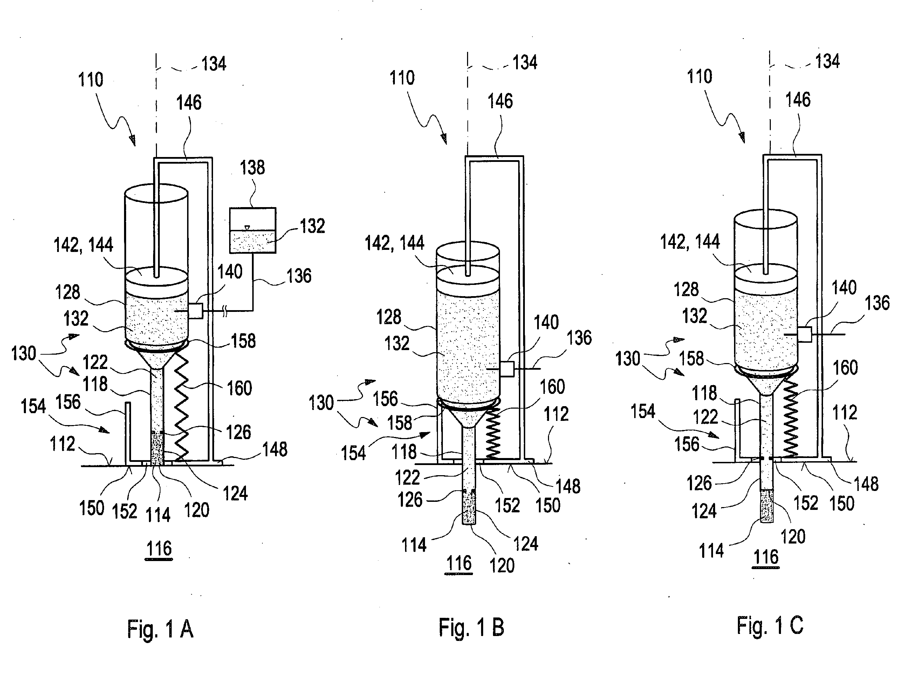

In den Figuren 1A bis 1C ist eine erfindungsgemäße Implantationsvorrichtung 110 in schematischer Darstellung von der Seite gezeigt. Gleichzeitig dienen die Figuren 1A bis 1C, welche unterschiedliche Stadien eines Implantationsvorgangs eines Sensorelements 114 durch eine Hautoberfläche 112 in ein Gewebe 116 zeigen, zur Erläuterung eines möglichen Ausführungsbeispiels eines Implantationsvorgangs mittels der Implantationsvorrichtung 110.

Die Implantationsvorrichtung 110 umfasst eine Kanüle 118 mit einer Implantationsspitze 120 an ihrem dem Gewebe 116 zuweisenden Ende. Diese Implantationsspitze 120 ist in den Figuren als stumpfe Implantationsspitze 120 ausgestaltet. Alternativ kann diese Implantationsspitze 120 jedoch auch mit einem scharfkantigen oder spitzen Element ausgestaltet sein, beispielsweise indem, wie dies bei vielen Kanülen üblich ist, die Implantationsspitze 120 angeschrägt und somit scharfkantig ausgebildet ist. Auf diese Weise ist kein separates Instrument zum Erzeugen einer Öffnung in der Hautoberfläche 112 erforderlich, sondern die Implantationsspitze 120 kann selbst die für ihr Eindringen in das Gewebe 116 erforderliche Öffnung in der Hautoberfläche 112 schaffen.

Die Kanüle 118 ist in dem einfachen in den Figuren dargestellten Ausführungsbeispiel als zylindrische Kanüle 118 dargestellt, mit einem zylindrischen Innenlumen 122. Auch andere Ausgestaltungen sind jedoch möglich.

An dem der Implantationsspitze 120 zuweisenden Ende des Innenlumens 122 ist ein Aufnahmebereich 124 vorgesehen. Dieser Aufnahmebereich 124 dient der Aufnahme des Sensorelements 114 und ist an seinem oberen, der Implantationsspitze 120 abgewandten Ende durch eine Verengung 126 in der Kanüle 118 begrenzt. Die Verengung 126 ist in dem dargestellten Ausführungsbeispiel als inwändige Sicke ausgebildet, also als eine nach innen ragende Verdickung der Wand der Kanüle 118. Auch andere Ausgestaltungen sind jedoch grundsätzlich möglich.

An ihrem oberen, der Implantationsspitze 120 abgewandten Ende ist die Kanüle 118 mit einem Hydraulikbehälter 128 verbunden. Dabei ist in den Figuren 1A bis 1C eine feste Verbindung zwischen Kanüle 118 und Hydraulikbehälter 128 vorgesehen, so dass der Hydraulikbehälter 128 und die Kanüle 118 gemeinsam eine Implantationseinheit 130 bilden, welche in dem dargestellten Ausführungsbeispiel als starre Implantationseinheit ausgebildet ist. Die Verbindung zwischen dem Hydraulikbehälter 128 und der Kanüle 118 ist dabei in dem dargestellten Ausführungsbeispiel trichterförmig ausgestaltet, so dass in dem Hydraulikbehälter 128 aufgenommene Hydraulikflüssigkeit 132, beispielsweise physiologische Kochsalzlösung, aus dem Hydraulikbehälter 128 leicht in die Kanüle 118 strömen kann. Auch andere Ausgestaltungen sind jedoch grundsätzlich möglich, beispielsweise eine Schlauchverbindung und/oder eine Rohrverbindung zwischen dem Hydraulikbehälter 128 und der Kanüle 118. Dabei sind in dem dargestellten Ausführungsbeispiel die Kanüle 118 und der Hydraulikbehälter 128 konzentrisch zu einer Achse 134 ausgerichtet. Auch diesbezüglich sind naturgemäß andere Ausgestaltungen möglich.

Der Hydraulikbehälter 128 ist über eine Verbindung 136 mit einem lediglich in Figur 1A und lediglich schematisch dargestellten Hydraulikvorrat 138 verbunden. Die Verbindung 136 umfasst dabei in dem dargestellten Ausführungsbeispiel ein Ventil 140, beispielsweise ein nach innen, ins Innere des Hydraulikbehälters 128 öffnendes Ventil, beispielsweise ein Rückschlagventil. Entsteht also ein Unterdruck im Inneren des Hydraulikbehälters 128, so öffnet dieses Ventil 140, und Hydraulikflüssigkeit 132 aus dem Hydraulikvorrat 138 kann in den Hydraulikbehälter 128 nachströmen.

Weiterhin umfasst die Implantationsvorrichtung 110 in dem dargestellten Ausführungsbeispiel eine Druckerzeugungsvorrichtung 142 zur Erzeugung eines Über- und/oder Unterdruckes innerhalb des Hydraulikbehälters 128. Diese Druckerzeugungsvorrichtung 142 umfasst in dem dargestellten Ausführungsbeispiel einen Druckkolben 144, welcher mit einem Kolbengestänge 146 verbunden ist. Dieses Kolbengestänge 146 wiederum ist mit einer Auflage 148 mit einer Auflagefläche 150 zur Auflage auf der Hautoberfläche 112 verbunden. Diese Auflage 148 weist eine Öffnung 152 auf, welche die Kanüle 118 ringförmig umgibt und durch welche die Kanüle 118 in das Gewebe 116 abgesenkt werden kann. Die Implantationseinheit 130 ist also als Ganzes relativ zum Druckkolben, dem Kolbengestänge 146 und der Auflage 148 axial, das heißt entlang der Achse 134, bewegbar.

Die Implantationsvorrichtung 110 in dem in den Figuren dargestellten Ausführungsbeispiel weist weiterhin eine Vorrichtung 154 zur Begrenzung und/oder Einstellung der Implantationstiefe auf. Die Vorrichtung 154 umfasst die Auflage 148 mit der Auflagefläche 150 und der Öffnung 152. Weiterhin umfasst die Vorrichtung 154 einen Tiefenanschlag 156, welcher beispielsweise senkrecht von der Auflage 148 absteht und als Stopper fungiert. Der Tiefenanschlag 156 ist, wie beispielsweise in Figur 1B erkennbar, eingerichtet, um an einem den Hydraulikbehälter 128 im trichterförmigen Übergangsbereich zur Kanüle 118 aufgenommenen Ringelement 158 anzuschlagen und dadurch die Eindringtiefe der Kanüle 118 in das Gewebe 116 zu begrenzen.

Weiterhin umfasst die Vorrichtung 154 bzw. die Implantationsvorrichtung 110 ein Federelement 160. Dieses Federelement 160, welches beispielsweise als Spiralfeder ausgestaltet sein kann, dient gleichzeitig als Rückholfeder und ist an einem Ende auf der Auflage 148 und an einem anderen Ende an dem Hydraulikbehälter 128 bzw. dem Ringelement 158 abgestützt. Auf diese Weise wird das Federelement 160 beim Eindringen der Kanüle 118 in das Gewebe 116 komprimiert, so dass das Eindringen der Kanüle 118 entgegen der Fedcrkraft dieses Federelements 160 erfolgen muss. Die Aufwärtsbewegung hingegen, bei welcher die Kanüle 118 wieder aus dem Gewebe 116 herausgezogen wird, erfolgt vorzugswcisc unterstützt durch die Federkraft des Federelements 160.

Anhand der Figurensequenz in den Figuren 1A bis 1C soll ein typischer Implantationsvorgang erläutert werden. Auch andere Ausgestaltungen des Implantationsvorganges bzw. der Implantationsvorrichtung 110 sind jedoch grundsätzlich möglich.

In dem in Figur 1A dargestellten Ausgangszustand befindet sich die Implantationseinheit 130 oberhalb der Hautoberfläche 112. Das Sensorelement 114 ist in dem Aufnahmebereich 124 aufgenommen. Diesbezüglich sei angemerkt, dass in diesem Zustand das Sensorelement 114 auch ganz oder teilweise erst erzeugt werden kann. Zu diesem Zweck kann beispielsweise die Kanüle 118 zumindest im Bereich des Aufnahmebereichs 124 als transparente Kanüle ausgestaltet sein. Anstelle von Stahl oder anderen metallischen Materialien lassen sich in diesem Fall beispielsweise transparente Kunststoffe, transparente Keramiken oder Glas als Kanülenmaterial einsetzen. Auch Kompositmaterialien sind möglich. Die Implantationsvorrichtung 110 kann beispielsweise eine in den Figuren nicht dargestellte Lichtquelle umfassen, welche den Aufnahmebereich 124 von außen beleuchtet, so dass eine Polymerisation des Sensorelements 114 in dem Aufnahmebereich 124 stattfinden kann. Alternativ kann jedoch auch ein bereits fertig hergestelltes Sensorelement 114 in den Aufnahmebereich 124 geladen werden, um dann die Implantationsvorrichtung 110 in den in Figur 1A dargestellten Ausgangszustand, in welchem die Auflage 148 auf der Hautoberflächc 112 aufliegt, zu bringen.

Anschließend wird die Implantationseinheit 130, wie in Figur 1B dargestellt, relativ zur Auflage 148 abgesenkt. Dieses Absenken kann beispielsweise manuell oder getrieben durch einen oder mehrere Aktuatoren erfolgen. Der Aktuator ist in den Figuren nicht dargestellt und kann beispielsweise zwischen die Auflage 148 und die Implantationseinheit 130 geschaltet werden, um eine Relativbewegung dieser Elemente 130, 148 anzutreiben. Die Ausgestaltung unter Verwendung mindestens eines Aktuators bietet den Vorteil, dass die Geschwindigkeit des Eindringens in das Gewebe 116 besonders gleichmäßig ausgestaltet werden kann, wodurch eine Gleichmäßigkeit der Implantation und eine möglichst schmerzfreie Implantation gewährleistet werden können. Wie oben beschrieben, kann zum Zweck des Eindringens der Kanüle 118 in das Gewebe 116 separat eine Öffnung in der Hautoberfläche 112 erzeugt werden, oder die Kanüle 118 kann selbst mit einem spitzen Ende ausgestaltet sein.

Das Absenken der Implantationseinheit 130 gemäß Figur 1B erfolgt so lange, bis das Ringelement 158 gegen den Tiefenanschlag 156 stößt. Der Tiefenanschlag 156 kann beispielsweise auch als verstellbarer Tiefenanschlag ausgestaltet sein, so dass die Eindringtiefe und damit die Implantationstiefe gezielt beeinflusst werden kann. Alternativ oder zusätzlich kann auch beispielsweise die Position des Ringelements 158 verändert werden, um eine Einstellung der Implantationstiefe vorzunehmen. Auch andere Möglichkeiten sind denkbar. Während des Absenkens verbleibt der Druckkolben 144 mittels des Kolbengestänges 146 vorzugsweise in einer fixierten Position relativ zur Hautoberfläche 112, so dass sich de facto dieser Druckkolben 144 innerhalb des Hydraulikbehälters 128 hebt und das der Hydraulikflüssigkeit 132 zur Verfügung stehende Volumen innerhalb des Hydraulikbehälters 128 anwächst. Hierdurch entsteht kurzfristig ein Unterdruck innerhalb des Hydraulikbehälters 128. Aufgrund dieses Unterdrucks öffnet das Rückschlagventil 140, und Hydraulikflüssigkeit 132 aus dem Hydraulikvorrat 138 kann ins Innere des Hydraulikbehälters 128 nachströmen.

Nach der in Figur 1B dargestellten Abwärtsbewegung entlang der Achse 134, bei welcher die Kanüle 118 in das Gewebe 116 eindringt, erfolgt anschließend gemäß Figur 1C eine Aufwärtsbewegung der Implantationseinheit 130, ebenfalls in Richtung der Achse 134. Dabei wird die Kanüle 118 aus dem Gewebe 116 herausgezogen. Wiederum verbleibt der Druckkolben 144 dabei in fester Position relativ zur Hautoberfläche 112, so dass sich wiederum die Implantationseinheit 130 relativ zu diesem Druckkolben 144 nach oben bewegt. Hierdurch steigt der Druck der Hydraulikflüssigkeit 132 im Inneren des Hydraulikbehälters 128 an, da nunmehr das Ventil 140 schließt. Durch diesen hydraulischen Druck wird das Sensorelement 114 aus dem Aufnahmebereich 124 der Kanüle 118 heraus in das Gewebe 116 geschoben und von der Kanüle 118 getrennt. Der Implantationsvorgang ist damit beendet.

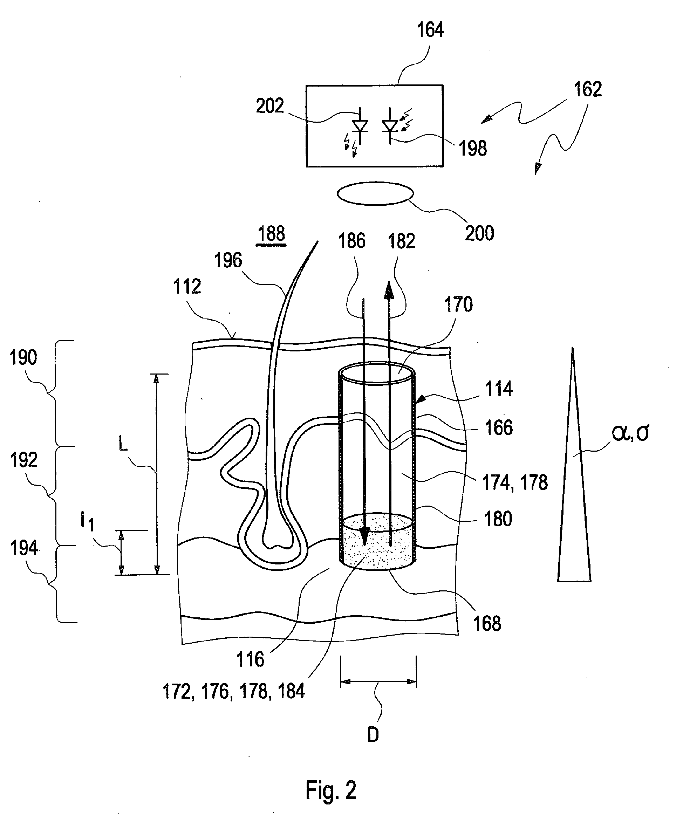

In Figur 2 ist ein Ausführungsbeispiel einer Sensoranordnung 162 dargestellt, welche einen mittels der Implantationsvorrichtung 110, beispielsweise gemäß dem Ausführungsbeispiel in den Figuren 1A bis 1C, implantierbares Sensorelement 114 sowie eine Detektionseinrichtung 164 umfasst. Grundsätzlich können mittels der Implantationsvorrichtung 110 eine Vielzahl von Sensorelementen 114 implantiert werden, wobei in Figur 2 lediglich ein spezielles Ausführungsbeispiel in implantiertem Zustand dargestellt ist. Das Sensorelement 114 weist einen einstückigen Formkörper 166 mit einem Sensorende 168 und einem Koppelende 170 auf. Der Formkörper 116 ist in diesem Ausführungsbeispiel beispielsweise als durchgehender Hydrogel-Formkörper ausgebildet. Der Formkörper 166 weist eine im Wesentlichen zylinderförmige Gestalt auf, mit einem Durchmesser D von ca. 200 bis 500 µm und einer Gesamtlänge L von ca. 2 bis 5 mm. Dabei ist das Sensorelement 114 unterteilt in einen Sensorbereich 172, welcher im implantierten Zustand ins Innere des Gewebes 116 hineinweist, und ein transparentes Koppelteil 174. Der Sensorbereich 172 weist eine Länge l₁ von ca. 200 bis 500 µm auf. In dem Sensorbereich 172 ist ein Sensormaterial 176 in ein Matrixmaterial 178 eingebettet, wobei das Matrixmaterial 178 beispielsweise auch im Bereich des Koppelteils 170 vorhanden sein kann. Das Matrixmaterial 178 kann beispielsweise ein transparentes Hydrogel umfassen.

Weiterhin ist in Figur 2 dargestellt, dass das Sensorelement 114 optional von einer Beschichtung 180 umgeben sein kann, beispielsweise einer biokompatiblen Beschichtung und/oder einer Beschichtung mit einem heilungsfördernden Wirkstoff. Die Beschichtung 180 kann beispielsweise in einem Layer-by-Layer-Verfahren und/oder einem Plasmabeschichtungsverfahren auf den Formkörper 166 aufgebracht werden.

Weiterhin ist in Figur 2 dargestellt, dass das transparente Koppelteil 174 als "Fenster" für die Auskopplung eines optischen Signals 182 dient. Dieses optische Signal 182 kann beispielsweise ein aus dem Sensormaterial 176 emittiertes und/oder reflektiertes Licht umfassen, wobei emittiertes Licht beispielsweise in Form von Fluoreszenzlicht und/oder Lumineszenzlicht emittiert werden kann. Dieses optische Signal 182 des Sensormaterials 176 ist vorzugsweise sensitiv auf die Anwesenheit eines Analyten in dem das Sensorende 168 umgebenden Körpergewebe 116. Weiterhin kann in dem Sensorbereich 172 neben dem Sensormaterial 176 noch ein Referenzmaterial 184 enthalten sein, welches ebenfalls zu dem optischen Signal 182 beitragen kann und eine Referenzkomponente dieses optischen Signals 182 reflektieren bzw. emittieren kann. Weiterhin ist in Figur 2 ein optionaler Anregungsstrahl 186 dargestellt, mittels dessen beispielsweise das Sensormaterial 176 und/oder das Referenzmaterial 184 spezifisch angeregt werden kann. Das Erfordernis der Verwendung eines derartigen Anregungsstrahls 186 ist abhängig von der Art des Sensormaterials 176 und/oder des Referenzmaterials 184 und/oder vom verwendeten optischen Nachweismechanismus zum Nachweis des mindestens einen Analyten in dem Körpergewebe 116 und/oder einer Körperflüssigkeit, welche den Sensorbereich 172 umgibt. Das Koppelteil 174 kann als Lichtwellenleiter dienen, kann jedoch auch als einfaches, homogenes und transparentes Fenster ohne Lichtwellenleitungseigenschaften ausgestaltet sein.

Das Koppelteil 174 wirkt dann lediglich als Fenster zur Betrachtung des Sensorbereichs 172 vom Außenbereich 188 außerhalb der Hautoberfläche 112.

Dabei ist in dem Ausführungsbeispiel gemäß Figur 2 zu erkennen, dass das Sensorelement 114 vorzugsweise derart in das Körpergewebe 116 implantiert ist, dass dessen Koppelende 170 noch unterhalb der Hautoberfläche 112 angeordnet ist. Die Hautoberfläche 112 oberhalb des Koppelendes 170 ist vorzugsweise im Messbetrieb bereits wieder ausgeheilt.

Als Beispiel eines Körpergewebes 116 ist in dem in Figur 2 dargestellten Ausführungsbeispiel eine Hautpartie mit einer Epidermis 190, einer Dermis 192 und einer Subcutis 194 gezeigt, wobei als Beispiel eines Größenvergleichs ein Haar 196 dargestellt ist. Symbolisch sind in Figur 2 weiterhin die Absorption α und die Streuung σ aufgetragen. Dabei ist zu erkennen, dass die Streuung σ und die Absorption α im Bereich der Hautoberfläche 112 gering sind und mit zunehmender Tiefe im Inneren des Körpergewebes 116 anwachsen. Es sei darauf hingewiesen, dass die dargestellte Hautpartie lediglich beispielhaft für einen Ort der Implantation zu verstehen ist, so dass eine Implantation auch in andere Arten von Körpergewebe 116 erfolgen kann, wie beispielsweise ein Gewebe innerhalb eines Auges oder auch in andere Arten von Körpergewebe.

Die Sensoranordnung 162 in dem Ausführungsbeispiel gemäß Figur 2 umfasst neben dem Sensorelement 114 die Detektionseinrichtung 164. Die Detektionseinrichtung 164 weist, sofern optische Nachweismethoden und optische Sensorelement 114 verwendet werden, beispielsweise mindestens einen optischen Detektor 198 auf. Werden andere Arten von Sensorelementen 114 verwendet, so können entsprechend andere Arten von Detektionseinrichtungen vorgesehen sein, beispielsweise elektrische Detektionsvorrichtungen, beispielsweise zum Detektieren einer Ladung, einer Spannung oder eines Stroms.

Der optische Detektor 198 ist in Figur 2 lediglich symbolisch dargestellt und hier als Photodiode symbolisiert. Es können jedoch eine Vielzahl optischer Detektoren und/oder zusätzlicher Vorrichtungen, beispielsweise Vorrichtungen zur spektralen Trennung des optischen Signals 182, vorgesehen sein, um das optische Signal 182 des Sensormaterials 176 und/oder des Referenzmaterials 184 optimal und gegebenenfalls spektral aufgelöst spezifisch zu erfassen. Die Detektionseinrichtung 164 ist dabei in Figur 2 derart ausgestaltet, dass an das Koppelende 170 des Sensorelements 114 angekoppelt werden kann, wobei die Kopplung vorzugsweise durch die obersten Schichten des Körpergewebes 116 hindurch erfolgen kann. Beispielsweise kann die Detektionseinrichtung 164 zu diesem Zweck auf die Hautoberfläche 112 aufgesetzt werden. Die Detektionseinrichtung 164 ist in Figur 2 optional mit zusätzlichen optischen Vorrichtungen 200 ausgestattet, welche ebenfalls Icdiglich symbolisch dargestellt sind und welche beispielsweise entsprechende Optiken wie zum Beispiel Linsen, Objektive, Blenden oder ähnliches umfassen kann.

Weiterhin umfasst die Detektionseinrichtung 164 in dem in Figur 2 dargestellten Ausführungsbeispiel optional mindestens eine Strahlenquelle 202 zur Erzeugung des optionalen Anregungsstrahls 186. Die Strahlenquelle 202 ist wiederum symbolisch als Leuchtdiode dargestellt, wobei jedoch eine Vielzahl anderer Arten von Strahlenquellen umfasst sein können.

Neben der optischen Vorrichtung 200, dem optischen Detektor 198 und der Strahlenquelle 202 kann die Detektionseinrichtung 164 weiterhin zusätzliche Komponenten enthalten, wie beispielsweise Ein- und Ausgabemittel, Energieversorgungen, Datenverarbeitungseinrichtungen oder ähnliches. Auf diese Weise kann optional das von dem implantierten Sensor 114 erzeugte Signal verarbeitet werden und beispielsweise in eine Analytkonzentration umgerechnet werden. Auch eine unmittelbare Anzeige dieser Analytkonzentration an einen Benutzer ist beispielsweise optional möglich.

### Bezugszeichenliste

- 110: Implantationsvorrichtung
- 112: Hautoberfläche
- 114: Sensorelement
- 116: Gewebe
- 118: Kanüle
- 120: Implantationsspitze
- 122: Innenlumen
- 124: Aufnahmebereich
- 126: Verengung
- 128: Hydraulikbehälter
- 130: Implantationseinheit
- 132: Hydraulikflüssigkeit
- 134: Achse
- 136: Verbindung
- 138: Hydraulikvorrat
- 140: Ventil
- 142: Druckerzeugungsvorrichtung
- 144: Druckkolben
- 146: Kolbengestänge
- 148: Auflage
- 150: Auflagefläche
- 152: Öffnung
- 154: Vorrichtung zur Einstellung und/oder Begrenzung der Implantationstiefe
- 156: Tiefenanschlag
- 158: Ringelement
- 160: Federelement
- 162: Sensoranordnung
- 164: Detektionseinrichtung
- 166: Formkörper
- 168: Sensorende
- 170: Koppelende
- 172: Sensorbereich
- 174: transparentes Koppelteil
- 176: Sensormaterial
- 178: Matrixmaterial
- 180: Beschichtung
- 182: optisches Signal
- 184: Referenzmaterial
- 186: Anregungsstrahl
- 188: Außenbereich
- 190: Epidermis
- 192: Dermis
- 194: Subcutis
- 196: Haar
- 198: optischer Detektor
- 200: optische Vorrichtungen
- 202: Strahlenquelle

## Patentansprüche

1. Implantationsvorrichtung (110) zur Implantation eines Sensorelements (114) zur Erfassung mindestens eines Analyten in einer Körperflüssigkeit oder einem Körpergewebe (116), umfassend mindestens eine Kanüle (118) zum Durchdringen einer Hautoberfläche (112) eines Patienten, wobei die Kanüle (118) mindestens einen Aufnahmebereich (124) zum Aufnehmen des Sensorelements (114) aufweist, wobei die Implantationsvorrichtung (110) weiterhin mindestens einen mit der Kanüle (118) verbundenen Hydraulikbehälter (128) zum Aufnehmen eines Hydraulikfluids (132) aufweist, wobei die Implantationsvorrichtung (110) weiterhin mindestens eine Druckerzeugungsvorrichtung (142) aufweist, wobei die Druckerzeugungsvorrichtung (142) eingerichtet ist, um das Hydraulikfluid (132) mit einem Druck zu beaufschlagen, wobei das Sensorelement (114) mittels des Hydraulikfluid (132) aus der Kanüle (118) in das Körpergewebe (116) transferierbar ist, wobei der Hydraulikbehälter (128) über mindestens eine Verbindung (136) mit einem Hydraulikvorrat (138) zur Aufnahme und Bereitstellung des Hydraulikfluids (132) verbunden ist, wobei die Verbindung (136) mindestens ein Ventil (140), insbesondere ein Rückschlagventil, umfasst.

2. Implantationsvorrichtung (110) nach dem vorhergehenden Anspruch, weiterhin umfassend mindestens eine Vorrichtung (154) zur Einstellung und/oder Begrenzung der Implantationstiefe, insbesondere einen Tiefenanschlag (156).

3. Implantationsvorrichtung (110) nach dem vorhergehenden Anspruch, wobei die Vorrichtung (154) zur Einstellung und/oder Begrenzung der Implantationstiefe eine Auflagefläche (150) zur Auflage auf der Hautoberfläche (112) umfasst, insbesondere eine die Kanüle (118) zumindest teilweise ringförmig umgebende Auflagefläche (150).

4. Implantationsvorrichtung (110) nach einem der beiden vorhergehenden Ansprüche, wobei die Kanüle (118) und der Hydraulikbehälter (128) eine Implantationseinheit (130) bilden, wobei die Vorrichtung (154) zur Einstellung und/oder Begrenzung der Implantationstiefe über mindestens ein Federelement (160) mit der Implantationseinheit (130) verbunden ist.

5. Implantationsvorrichtung (110) nach einem der vorhergehenden Anspruch, wobei das Ventil (140) eingerichtet ist, um bei einem Unterdruck in dem Hydraulikbehälter (128) zu öffnen und ein Nachströmen von Hydraulikfluid (132) in den Hydraulikbehälter (128) zu ermöglichen.

6. Implantationsvorrichtung (110) nach einem der vorhergehenden Ansprüche, wobei die Druckerzeugungsvorrichtung (142) mindestens einen Druckkolben (144), insbesondere einen in dem Hydraulikbehälter (128) aufgenommenen Druckkolben (144), umfasst.

7. Implantationsvorrichtung (110) nach dem vorhergehenden Anspruch, wobei die Kanüle (118) und der Hydraulikbehälter (128) eine Implantationseinheit (130) bilden, wobei die Implantationseinheit (130) relativ zu dem Druckkolben (144) axial entlang einer Achse (134) der Implantationsvorrichtung (110) bewegbar ist.

8. Implantationsvorrichtung (110) nach einem der beiden vorhergehenden Ansprüche, wobei eine Position des Druckkolbens (144) relativ zu der Hautoberfläche (112) fixierbar ist, wobei der Druckkolben (144) mit mindestens einer Auflagefläche (150), insbesondere der Auflagefläche (150) der Vorrichtung (154) zur Einstellung und/oder Begrenzung der Implantationstiefe, verbunden ist, wobei die Verbindung insbesondere über ein Kolbengestänge (146) erfolgt.

9. Implantationsvorrichtung (110) nach einem der vorhergehenden Ansprüche, wobei das Hydraulikfluid (132) eine Kochsalzlösung, insbesondere eine physiologische Kochsalzlösung, umfasst.

10. Implantationsvorrichtung (110) nach einem der vorhergehenden Ansprüche, wobei die Kanüle (118) zumindest teilweise transparent für Licht, insbesondere Licht im ultravioletten Spektralbereich, ist.

11. Implantationsvorrichtung (110) nach dem vorhergehenden Anspruch, weiterhin umfassend mindestens eine Lichtquelle zum Vernetzen mindestens eines vernetzbaren Materials des Sensorelements (114) in der Kanüle (118).

12. Implantationsvorrichtung (110) nach einem der vorhergehenden Ansprüche, wobei die Kanüle (118) mindestens eine Verengung (126) zur Begrenzung des Aufnahmebereichs (124) aufweist.

13. Implantationsvorrichtung (110) nach einem der vorhergehenden Ansprüche, wobei die Implantationsvorrichtung (110) weiterhin eingerichtet ist, um ein in dem Körpergewebe (116) implantiertes Sensorelement (114) zu entfernen, wobei die Druckerzeugungsvorrichtung (142) eingerichtet ist, um einen Unterdruck in dem Hydraulikfluid (132) zu erzeugen, wobei das Sensorelement (114) in die Kanüle (118) saugbar ist.

14. Implantationsvorrichtung (110) nach einem der vorhergehenden Ansprüche, weiterhin umfassend mindestens ein Sensorelement (114) zur Erfassung mindestens eines Analyten in einer Körperflüssigkeit oder einem Körpergewebe (116).

## Claims

1. Implantation device (110) for implanting a sensor element (114) for detecting at least one analyte in a bodily fluid or body tissue (116), comprising at least one cannula (118) for piercing a skin surface (112) of a patient, wherein the cannula (118) has at least one holding area (124) for holding the sensor element (114), wherein the implantation device (110) furthermore has at least one hydraulic container (128), connected to the cannula (118), for holding a hydraulic fluid (132), wherein the implantation device (110) furthermore has at least one pressure generation device (142), wherein the pressure generation device (142) is designed to apply pressure to the hydraulic fluid (132), wherein the sensor element (114) can be transferred from the cannula (118) into the body tissue (116) using the hydraulic fluid (132), wherein the hydraulic container (128) is connected to a hydraulic reservoir (138) for holding and providing the hydraulic fluid (132) via at least one connection (136), and wherein the connection (136) comprises at least one valve (140), in particular a check valve.

2. Implantation device (110) according to the preceding claim, furthermore comprising at least one device (154) for setting and/or restricting the implantation depth, in particular a depth stop (156).

3. Implantation device (110) according to the preceding claim, wherein the device (154) for setting and/or restricting the implantation depth comprises a resting surface (150) for resting on the skin surface (112), in particular a resting surface (150) which at least partly annularly surrounds the cannula (118).

4. Implantation device (110) according to one of the two preceding claims, wherein the cannula (118) and the hydraulic container (128) form an implantation unit (130), with the device (154) for setting and/or restricting the implantation depth being connected to the implantation unit (130) via at least one spring element (160).

5. Implantation device (110) according to one of the preceding claims, wherein the valve (140) is designed to open in the case of negative pressure in the hydraulic container (128) and permit subsequent flow of hydraulic fluid (132) into the hydraulic container (128).

6. Implantation device (110) according to one of the preceding claims, wherein the pressure generation device (142) comprises at least one pressure piston (144), in particular a pressure piston (144) held in the hydraulic container (128).

7. Implantation device (110) according to the preceding claim, wherein the cannula (118) and the hydraulic container (128) form an implantation unit (130), with the implantation unit (130) being movable axially along an axis (134) of the implantation device (110), relative to the pressure piston (144).

8. Implantation device (110) according to one of the two preceding claims, wherein a position of the pressure piston (144) can be fixed relative to the skin surface (112), wherein the pressure piston (144) is connected to at least one resting surface (150), in particular the resting surface (150) of the device (154) for setting and/or restricting the implantation depth, the connection being effected in particular by means of a piston rod (146).

9. Implantation device (110) according to one of the preceding claims, wherein the hydraulic fluid (132) comprises a saline, in particular a physiological saline.

10. Implantation device (110) according to one of the preceding claims, wherein the cannula (118) is at least partly transparent to light, in particular light in the ultraviolet spectral range.

11. Implantation device (110) according to the preceding claim, furthermore comprising at least one light source for crosslinking, in the cannula (118), at least one material of the sensor element (114) which can be crosslinked.

12. Implantation device (110) according to one of the preceding claims, wherein the cannula (118) has at least one constriction (126) for limiting the holding area (124).

13. Implantation device (110) according to one of the preceding claims, wherein the implantation device (110) is furthermore designed to remove a sensor element (114) implanted in the body tissue (116), wherein the pressure generation device (142) is designed to generate negative pressure in the hydraulic fluid (132), with it being possible for the sensor element (114) to be sucked into the cannula (118).

14. Implantation device (110) according to one of the preceding claims, furthermore comprising at least one sensor element (114) for detecting at least one analyte in a bodily fluid or body tissue (116).

## Revendications

1. Dispositif d'implantation (110) pour l'implantation d'un élément de capteur (114) pour détecter au moins un analyte dans un liquide corporel ou un tissu corporel (116), comprenant au moins une canule (118) destinée à pénétrer à travers la surface de la peau (112) d'un patient, la canule (118) présentant au moins une région de réception (124) pour recevoir l'élément de capteur (114), le dispositif d'implantation (110) présentant en outre au moins un récipient hydraulique (128) connecté à la canule (118) pour recevoir un fluide hydraulique (132), le dispositif d'implantation (110) présentant en outre au moins un dispositif de génération de pression (142), le dispositif de génération de pression (142) étant prévu pour solliciter le fluide hydraulique (132) avec une pression, l'élément de capteur (114) pouvant être transféré au moyen du fluide hydraulique (132) hors de la canule (118) dans le tissu corporel (116), le récipient hydraulique (128) étant connecté par le biais d'au moins une connexion (136) à une réserve hydraulique (138) pour recevoir et fournir le fluide hydraulique (132), la connexion (136) comprenant au moins une soupape (140), notamment un clapet anti-retour.

2. Dispositif d'implantation (110) selon la revendication précédente, comprenant en outre au moins un dispositif (154) pour ajuster et/ou limiter la profondeur d'implantation, notamment une butée de profondeur (156).

3. Dispositif d'implantation (110) selon la revendication précédente, dans lequel le dispositif (154) pour ajuster et/ou limiter la profondeur d'implantation comprend une surface d'appui (150) à appuyer contre la surface de la peau (112), notamment une surface d'appui (150) entourant au moins en partie de manière annulaire la canule (118).

4. Dispositif d'implantation (110) selon l'une quelconque des deux revendications précédentes, dans lequel la canule (118) et le récipient hydraulique (128) forment une unité d'implantation (130), le dispositif (154) pour ajuster et/ou limiter la profondeur d'implantation étant connecté à l'unité d'implantation (130) par le biais d'au moins un élément à ressort (160).

5. Dispositif d'implantation (110) selon l'une quelconque des revendications précédentes, dans lequel la soupape (140) est prévue pour s'ouvrir dans le cas d'une dépression dans le récipient hydraulique (128) et pour permettre un reflux de fluide hydraulique (132) dans le récipient hydraulique (128).

6. Dispositif d'implantation (110) selon l'une quelconque des revendications précédentes, dans lequel le dispositif de génération de pression (142) comprend au moins un piston de pression (144), notamment un piston de pression (144) reçu dans le récipient hydraulique (128).

7. Dispositif d'implantation (110) selon la revendication précédente, dans lequel la canule (118) et le récipient hydraulique (128) forment une unité d'implantation (130), l'unité d'implantation (130) pouvant être déplacée par rapport au piston de pression (144) axialement le long d'un axe (134) du dispositif d'implantation (110).

8. Dispositif d'implantation (110) selon l'une quelconque des deux revendications précédentes, dans lequel une position du piston de pression (144) par rapport à la surface de la peau (112) peut être fixée, le piston de pression (144) étant connecté à au moins une surface d'appui (150), notamment la surface d'appui (150) du dispositif (154) pour ajuster et/ou limiter la profondeur d'implantation, la connexion étant réalisée notamment par une tringle de piston (146).

9. Dispositif d'implantation (110) selon l'une quelconque des revendications précédentes, dans lequel le fluide hydraulique (132) comprend une solution saline, notamment une solution de sel physiologique.

10. Dispositif d'implantation (110) selon l'une quelconque des revendications précédentes, dans lequel la canule (118) est au moins partiellement transparente à la lumière, en particulier la lumière dans la plage spectrale ultraviolette.

11. Dispositif d'implantation (110) selon la revendication précédente, comprenant en outre au moins une source de lumière pour réticuler au moins un matériau réticulable de l'élément de capteur (114) dans la canule (118).

12. Dispositif d'implantation (110) selon l'une quelconque des revendications précédentes, dans lequel la canule (118) présente au moins un rétrécissement (126) pour limiter la région de réception (124).

13. Dispositif d'implantation (110) selon l'une quelconque des revendications précédentes, dans lequel le dispositif d'implantation (110) est en outre prévu pour enlever un élément de capteur (114) implanté dans le tissu corporel (116), le dispositif de génération de pression (142) étant prévu pour produire une dépression dans le fluide hydraulique (132), l'élément de capteur (114) pouvant être aspiré dans la canule (118).

14. Dispositif d'implantation (110) selon l'une quelconque des revendications précédentes, comprenant en outre au moins un élément de capteur (114) pour détecter au moins un analyte dans un liquide corporel ou un tissu corporel (116).
